# FASCICULE DE BREVET EUROPEEN

(11) **EP 1 114 636 B1**
(45) Date de publication et mention de la délivrance du brevet: **20.04.2005**
(21) Numéro de dépôt: 00403575.4
(22) Date de dépôt: 18.12.2000
(51) Int. Cl.: A61K 7/027

(54) **Composition longue tenue structurée par un polymère et un corps gras pâteux**
Strukturierte langhaltige Zubereitung enthaltend ein Polymer und pastöse Fettstoffe
Structured long-wearing composition containing a polymer and a pasty fatty material

(30) Priorité: 28.12.1999 FR 9916588; 25.01.2000 FR 0001004
(43) Date de publication de la demande: 11.07.2001
(73) Titulaire: L'OREAL, 75008 Paris (FR)
(72) Inventeur: Ferrari, Véronique, 94700 Maisons-Alfort (FR); Jacques, Véronique, 94240 L'Hay-les-Roses (FR)
(74) Mandataire: Lhoste, Catherine

(56) Documents cités:
- EP-A- 0 923 928
- EP-A- 0 925 780
- WO-A-98/17243
- US-A- 3 148 125
- US-A- 5 500 209
- US-A- 5 783 657

## Description

La présente invention se rapporte à une composition de soin et/ou de traitement et/ou de maquillage de la peau, y compris du cuir chevelu, et/ou des lèvres des êtres humains, contenant une phase grasse liquide, structurée par un polymère particulier et un corps gras pâteux, se présentant notamment sous forme d'un stick de maquillage comme les rouges à lèvres, dont l'application conduit à un dépôt brillant, non-migrant et de longue tenue.

Dans les produits cosmétiques ou dermatologiques, il est courant de trouver une phase grasse liquide structurée, à savoir gélifiée et/ou rigidifiée ; ceci est notamment le cas dans les compositions solides comme les déodorants, les baumes et les rouges à lèvres, les produits anti-cerne et les fonds de teint coulés. Cette structuration est obtenue à l'aide de cires ou de charges. Malheureusement, ces cires et charges ont tendance à matifier la composition, ce qui n'est pas toujours souhaitable en particulier pour un rouge à lèvres ; en effet, les femmes sont toujours à la recherche d'un rouge à lèvres sous forme d'un bâton déposant un film de plus en plus brillant.

WO-A-98 17243 divulgue de gels de polyamide à terminaison ester.

Par « phase grasse liquide », au sens de l'invention, on entend une phase grasse liquide à température ambiante (25°C) et pression atmosphérique (760 mm de Hg), composée d'un ou plusieurs corps gras liquides à température ambiante, appelés aussi huiles, généralement compatibles entre eux.

Par corps gras, on entend un milieu non aqueux, non miscibles à l'eau et plus spécialement un composé hydrocarboné comportant une ou plusieurs chaînes carbonées ayant chacune au moins 5 atomes de carbone et pouvant comporter des groupes polaires comme un groupe acide carboxylique, un hydroxyle, ou polyol, amine, amide, acide phosphorique, phosphate, ester, éther, urée, carbamate, thiol, thioéther, thioester ; un composé siliconé comportant éventuellement des chaînes carbonées en bout ou pendantes, ces chaînes étant éventuellement substituées par un groupement fluoré ou perfluoré, (poly)amino acide, éther, hydroxyle, amine, acide, ester ; ou un composé fluoré ou perfluoré comme les hydrocarbures fluorés ou perfluorés ayant au moins 5 atomes de carbone, pouvant comporter un hétéroatome comme N, O, S, P et éventuellement une ou plusieurs fonctions polaires, comme un groupe éther, ester, amine, acide, carbamate, urée, thiol, hydroxyle.

La structuration de la phase grasse liquide permet en particulier de limiter son exsudation à température ambiante des compositions solides notamment dans des régions chaudes et humides et, en plus, de limiter, après dépôt sur la peau ou les lèvres, la migration de cette phase dans les rides et ridules (en dehors du tracé original), ce qui est particulièrement recherché pour un rouge à lèvres ou un fard à paupières. En effet, une migration importante de la phase grasse liquide, en particulier lorsqu'elle est chargée de matières colorantes, conduit à un effet inesthétique autour des lèvres et des yeux, accentuant particulièrement les rides et les ridules. Cette migration est souvent citée par les femmes comme un défaut majeur des rouges à lèvres et fards à paupières classiques.

La brillance est liée pour l'essentiel à la nature de la phase grasse liquide. Ainsi, il est possible de diminuer le taux de cires et de charges de la composition pour augmenter la brillance d'un rouge à lèvres mais alors, la migration de la phase grasse liquide augmente. Autrement dit, les taux de cires et de charges nécessaires à la réalisation d'un stick de dureté convenable sont un frein à la brillance du dépôt.

Le demandeur a trouvé que la perte de brillance d'un stick contenant des cires était liée à la structure cristalline anisotrope de ces composés. Il a donc envisagé la fabrication d'un stick, en réduisant le taux de cire et/ou de charges.

Par ailleurs, il est souhaitable que la composition présente une longue tenue dans le temps, notamment en ce qui concerne la couleur. Une mauvaise tenue se caractérise par une modification de la couleur (virage, palissement) généralement par suite d'une interaction avec le sébum et/ou la sueur de la peau et, pour les lèvres, d'une interaction avec la salive. En effet, une composition qui ne tient pas dans le temps oblige l'utilisateur à se maquiller très souvent. Or à ce jour, les utilisateurs souhaitent embellir leur visage et leur corps en y passant le moins de temps possible. Enfin, une composition de soin ou de maquillage doit être confortable à porter, à savoir non desséchante et ne tiraillant pas.

L'invention a justement pour objet une composition de soin et/ou de maquillage et/ou de traitement de la peau et/ou des lèvres du visage permettant de remédier à ces inconvénients.

De façon surprenante, le demandeur a trouvé que l'utilisation de polymères particuliers et de corps gras pâteux permettait de structurer, même en l'absence de cire, les phases grasses liquides sous forme de stick dont l'application sur les lèvres conduisait à un film brillant, non migrant, confortable, de longue tenue et n'exsudant pas à température ambiante.

L'invention s'applique non seulement aux produits de maquillage des lèvres mais aussi aux produits de soin et/ou de traitement de la peau, y compris du cuir chevelu, et des lèvres, comme les produits notamment en stick de protection solaire de la peau du visage, aux produits de maquillage de la peau, aussi bien du visage que du corps humain, comme les fonds de teints notamment coulés en stick ou en coupelle, les produits anti-cerne, les fards à paupières et les produits de tatouage éphémère, aux produits d'hygiène corporelle comme les déodorants notamment en stick, les shampooings et après-shampooings et aux produits de maquillage des yeux comme les eye-liners en particulier sous forme de crayon et les mascaras plus spécialement sous forme de pains et aussi les produits de soin du visage et du corps.

De façon plus précise, l'invention a pour objet une composition structurée contenant une phase grasse liquide, structurée par au moins un corps gras pâteux et au moins un polymère de masse moléculaire moyenne en poids inférieure à 100 000 et notamment inférieure à 50 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées ayant de 8 à 120 et mieux de 12 à 120 atomes de carbone, liées à ces motifs, ces chaînes grasses représentant de 40 à 98 % du nombre total des motifs à hétéroatome et des chaînes grasses, la phase grasse liquide, le corps gras pâteux et le polymère formant un milieu physiologiquement acceptable, le corps gras pâteux ayant un point de fusion allant de 20 à 55 °C, et/ou une viscosité à 40 °C allant de 0,1 à 40 Pa.s mesurée au Contraves TV ou Rhéomat 80, équipé d'un mobile toumant à 240 min⁻¹.

Par au "moins une chaîne grasse", on entend une ou plusieurs chaînes grasses. Ces chaînes grasses peuvent être liées directement au squelette polymérique ou via un groupement polaire notamment du type acide carboxylique, hydroxyle, polyol, amine, amide, acide phosphorique, phosphate, ester, éther, urée, carbamate, thiol, thioéther, thioester.

La composition de l'invention peut se présenter sous forme de pâte, de solide, de crème plus ou moins visqueuse. Elle peut être une émulsion simple ou multiple notamment huile-dans-eau ou eau-dans-huile, un gel notamment anhydre rigide ou souple. En particulier, elle se présente sous coulée en stick ou en coupelle et plus spécialement sous forme d'une composition à phase continue ou externe grasse, et en particulier, d'un gel rigide anhydre notamment de stick anhydre. Avantageusement la composition de l'invention est autoportée. La composition est en particulier un fond de teint, un produit anti cernes, un produit à lèvres notamment un rouge à lèvres, un fard à paupières ou à joues, un produit de maquillage du corps, un eye liner ou un mascara, ou encore un produit de soin hydratant, nourrissant ou de protection.

La gélification des huiles est modulable selon la nature du polymère à hétéroatome utilisé, et peut être telle que l'on obtienne une structure rigide sous forme d'un bâton ou d'un stick. Ces bâtons lorsqu'ils sont colorés permettent, après application, d'obtenir un dépôt brillant, homogène en couleur, ne migrant pas dans les rides et ridules de la peau, entourant en particulier les lèvres, mais aussi les yeux, et de longue tenue.

De façon avantageuse, le polymère de la composition de l'invention comprend une masse moléculaire moyenne en poids allant de 1 000 à 30 000 et mieux de 1 000 à 10 000 et encore mieux de 2000 à 8000.

Le polymère structurant de la composition de l'invention est un solide non déformable à température ambiante (25°C) et pression atmosphérique (760mm de Hg). Il est capable de structurer la composition sans l'opacifier.

Par "chaînes fonctionnalisées" au sens de l'invention, on entend une chaîne alkyle comportant un ou plusieurs groupes fonctionnels ou réactifs notamment choisis parmi les groupes hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Par "polymère", on entend au sens de l'invention un composé ayant au moins 2 motifs de répétition et mieux au moins 3 motifs identiques.

Par "motifs de répétition hydrocarbonés", on entend au sens de l'invention un motif ou chaînon comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs ou chaînons comprennent, en outre, chacun un ou plusieurs hétéroatomes non pendants et se trouvant dans le squelette polymérique. Dans la suite du texte, on utilisera indifféremment les termes chaînon ou motif.

En outre, le polymère de la composition de l'invention comprend de 40 à 98 % de chaînes grasses par rapport au nombre total des chaînons à hétéroatome et des chaînes grasses et mieux de 50 à 95 %. La nature et la proportion des motifs (ou chaînons) à hétéroatome est fonction de la nature de la phase grasse liquide et est en particulier similaire à la nature de la phase grasse. Ainsi, plus les motifs (ou chaînons) à hétéroatome sont polaires et en proportion élevée dans le polymère, ce qui correspond à la présence de plusieurs hétéroatomes, plus le polymère a de l'affinité avec les huiles polaires. En revanche, plus les motifs (ou chaînons) à hétéroatome sont peu polaires voire apolaires ou en proportion faible, plus le polymère a de l'affinité avec les huiles apolaires.

Les chaînons ou motifs à hétéroatome comprennent chacun de un à plusieurs hétéroatomes choisis parmi les atomes d'azote, de soufre, de phosphore et leurs associations, associés éventuellement à un ou plusieurs atomes d'oxygène. Ces chaînons peuvent notamment comprendre un groupement polaire du type carbonyle. De préférence, ces chaînons comportent comme hétératomes des atomes d'azote, ces atomes d'azote étant avantageusement non pendants.

Les chaînons ou motifs à hétéroatome sont en particulier des chaînons comportant des chaînons hydrocarbonés et des chaînons siliconés formant un squelette organopolysiloxane, des chaînons amide formant un squelette du type polyamide, des chaînons "isocyanates" ou plus exactement carbamate et/ou urée formant un squelette polyuréthane, polyurée et/ou polyurée-uréthane. De préférence, ces chaînons sont des chaînons amide. Avantageusement, les chaînes pendantes sont liées directement à l'un au moins des hétéroatomes du squelette polymérique, et en particulier à l'un au moins des atomes d'azote des chaînons amide.

Entre les motifs hydrocarbonés, le polymère peut comprendre des motifs oxyalkylénés.

Comme polymères structurant préférés utilisables dans l'invention, on peut citer les polyamides ramifiés par des chaînes grasses pendantes et/ou terminales ayant de 8 à 120 et en particulier, de 12 à 120 atomes de carbone, et notamment, de 12 à 68 atomes de carbone, la ou les chaînes grasses pendantes et/ou la ou les chaînes grasses terminales étant liées au squelette polyamide par des fonctions ester.

Ces polymères sont de préférence des polymères résultant d'une polycondensation entre un diacide carboxylique à au moins 32 atomes de carbone (ayant notamment de 32 à 44 atomes de carbone) avec une diamine ayant au moins 2 atomes de carbone (ayant notamment de 2 à 36 atomes de carbone). Le diacide est de préférence un dimère d'acide gras ayant au moins 16 atomes de carbone comme l'acide oléïque, linoléïque, linolénïque. La diamine est de préférence l'éthylène diamine, l'hexylène diamine, l'hexaméthylène diamine, le phénylène diamine, l'éthylène triamine et encore mieux l'éthylène diamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple à 18 atomes de carbone.

Ces polymères sont plus spécialement ceux décrits dans le document US-A-5783657 de la société Union Camp. Chacun de ces polymères satisfait notamment la formule (l) suivante : 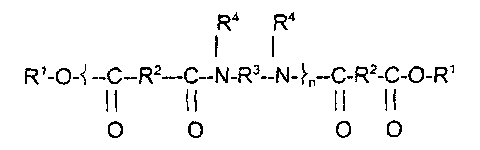 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvu d'au moins 1 et mieux d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

Dans le cas particulier de la formule (I), les chaînes grasses terminales éventuellement fonctionnalisées au sens de l'invention sont les chaînes terminales liées au dernier hétéroatome, ici azote, du squelette polyamide.

En particulier, les groupes ester de la formule (l), qui font partie des chaînes grasses terminales ou pendantes au sens de l'invention, représentent de 15 à 40 % du nombre total des groupes ester et amide et mieux de 20 à 35 %. De plus, n représente avantageusement un nombre entier allant de 1 à 5 et mieux supérieur à 2. De préférence, R¹ est un groupe alkyle en C₁₂ à C₂₂ et de préférence en C₁₆ à C₂₂. Avantageusement, R² peut être un groupe hydrocarboné (alkylène notamment) en C₁₀ à C₄₂ ayant une structure d'acide gras polymérisé ou de dimère dont les groupements acide carboxylique ont été enlevés (ces groupements servant à la formation de l'amide). De préférence, 50 % au moins et mieux 75 % des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

Selon l'invention, la structuration de la phase grasse liquide est obtenue à l'aide d'un ou plusieurs polymères de formule (I). En général, les polymères de formule (I) se présentent sous forme de mélanges de polymères, ces mélanges pouvant en outre contenir un produit de synthèse correspondant à un composé de formule (I) où n vaut 0, c'est-à-dire un diester.

A titre d'exemple de polymères structurant utilisables dans la composition selon l'invention, on peut citer les produits commerciaux vendus par la société Bush Boake Allen sous les noms Uniclear 80 et Uniclear 100. lls sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en C₃₆ condensé sur l'éthylène diamine, de masse moléculaire moyenne d'environ 6000. Les groupes esters terminaux résultent de l'estérification des terminaisons d'acide restantes par l'alcool cétylique, stéarylique ou leurs mélanges (appelés aussi alcool cétylstéarylique).

Les polymères structurant de la composition ont avantageusement une température de ramollissement supérieure à 65°C et notamment 70°C et pouvant aller jusqu'à 190°C. De préférence, ils présentent une température de ramollissement allant de 80 à 130°C et mieux de 80°C à 105°C. Cette température de ramollissement est plus basse que celle des polymères structurant connus, ce qui facilite la mise en oeuvre des polymères objet de l'invention et limite les détériorations de la phase grasse liquide.

Les polymères objet de l'invention présentent du fait de leur (s) chaîne (s) grasse (s), une bonne solubilité dans les huiles (à savoir composés liquides, non miscibles à l'eau) et donc conduisent à des compositions macroscopiquement homogènes même avec un taux élevé (au moins 25%) de polymère, contrairement aux polymères de l'art antérieur exempts de chaîne grasse.

Avantageusement, le polymère est associé à au moins un composé amphiphile liquide à température ambiante, de valeur de balance hydrophile/lipophile (HLB) inférieure à 12 et mieux inférieure à 8 et encore mieux allant de 1 à 7 et de préférence de 1 à 5 et mieux de 3 à 5. Selon l'invention, on peut utiliser un ou plusieurs composés amphiphiles. Ces composés amphiphiles ont pour but de renforcer les propriétés structurantes du polymère à hétéroatome, de faciliter la mise en oeuvre du polymère et d'améliorer la capacité à déposer notamment lorsque la composition est sous forme de stick.

Le ou les composés amphiphiles utilisables dans la composition de l'invention comprennent une partie lipophile liée à une partie polaire, la partie lipophile comportant une chaîne carbonée ayant au moins 8 atomes de carbone notamment, de 16 à 32 atomes de carbone et mieux de 18 à 28 atomes de carbone. De préférence, la partie polaire de ce ou ces composés amphiphiles est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés. En particulier, le composé amphiphile est un ester choisi parmi les hydroxystéarates, les oléates, les isostéarates du glycérol, du sorbitan ou du méthylglucose, ou encore les alcools gras ramifiés en C₁₂ à C₂₆ comme l'octyldodécanol et leurs mélanges. Parmi les esters, on préfère les monoesters et les mélanges de mono- et de di-esters.

Le taux de composé amphiphile et celui du polymère à hétéroatome sont choisis selon la viscosité ou dureté désirée pour la composition et en fonction de l'application particulière envisagée. Les quantités respectives de polymère et de composé amphiphile doivent notamment être telles qu'elles permettent l'obtention d'un stick délitable présentant notamment une dureté allant de 20 à 2 000 g en particulier de 20 à 1 500 g et mieux de 20 à 900 g, par exemple de 50 à 600 g ou encore mieux de 150 à 450 g. Cette dureté peut être mesurée selon une méthode de pénétration d'une sonde dans ladite composition et en particulier à l'aide d'un analyseur de texture (par exempleTA-XT2i; de chez Rhéo) équipé d'un cylindre en ébonite de 25 mm de haut et 8 mm de diamètre. La mesure de dureté est effectuée à 20°C au centre de 5 échantillons de la dite composition. Le cylindre est introduit dans chaque échantillon de composition à une pré-vitesse de 2mm/s puis à une vitesse de 0,5 mm/s et enfin à une post-vitesse de 2mm/s, le déplacement total étant de 1mm. La valeur relevée de la dureté est celle du pic maximum. L'erreur de mesure est de +/- 50 g.

La dureté de la composition peut aussi être mesurée par la méthode dite du fil à couper le beurre, qui consiste à couper un bâton de rouge à lèvres de 8,1 mm et à mesurer la dureté à 20°C, au moyen d'un dynamomètre DFGHS 2 de la société Indelco-Chatillon se déplaçant à une vitesse de 100mm/minute. Elle est exprimée comme la force de cisaillement (exprimée en gramme) nécessaire pour couper un stick dans ces conditions. Selon cette méthode la dureté d'une composition en stick selon l'invention va de 30 à La dureté de la composition selon l'invention est telle que la composition est avantageusement autoportée et peut se déliter aisément pour former un dépôt satisfaisant sur la peau et les lèvres. En outre, avec cette dureté, la composition de l'invention résiste bien aux chocs.

Selon l'invention, la composition sous forme de stick a le comportement d'un solide élastique déformable et souple, conférant à l'application une douceur élastique remarquable. Les compositions en stick de l'art antérieur n'ont pas cette propriété d'élasticité et de souplesse.

En pratique, la quantité de polymère représente de 0,5 à 80 % du poids total de la composition, de préférence de 2 à 60 % et mieux de 5 à 40 %. La quantité de composé amphiphile représente en pratique de 0,1 % à 35 % du poids total de la composition, par exemple de 1 % à 20 % et mieux de 2 % à 15 %, s'il est présent.

Avantageusement, la phase grasse liquide de la composition contient une ou plusieurs huile(s) liquide(s) dont la nature chimique est voisine de celle du squelette du polymère structurant (hydrocarboné ou siliconé) En particulier, la phase grasse liquide structurée par un squelette de type polyamide contient une huile ou un mélange d'huiles liquides apolaires notamment hydrocarbonées.

Pour une phase grasse liquide structurée par un polymère à squelette partiellement siliconé, cette phase grasse contient, de préférence une huile ou un mélange d'huiles liquides siliconées.

Pour une phase grasse liquide structurée par un polymère apolaire du type hydrocarboné, cette phase grasse contient avantageusement une huile ou un mélange d'huiles apolaires liquides, notamment hydrocarbonées. Par huile hydrocarbonnée, on entend au sens de l'invention des huiles contenant principalement des atomes de carbone et d'hydrogène.

En particulier, les huiles polaires de l'invention sont :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse de formule R₅COOR₆ dans laquelle R₅ représente le reste d'un acide gras supérieur linéaire ou ramifié comportant de 7 à 30 atomes de carbone et R₆ représente une chaîne hydrocarbonée ramifiée contenant de 3 à 30 atomes de carbone comme par exemple l'huile de Purcellin (octanoate de cétostéaryle), l'isononanoate d'isononyle, le benzoate d'alcool en C₁₂ à C₁₅ ;
- les esters et les éthers de synthèse comme le myristate d'isopropyle, le palmitate d'éthyl-2-hexyle, des octanoates, décanoates ou ricinoléates d'alcools ou de polyalcools ;
- les alcools gras en C₈ à C₂₆ comme l'alcool oléique ;
- les acides gras en C₈ à C₂₆ comme l'acide oléique, linolénique ou linoléique ;
- leurs mélanges.

Les huiles apolaires selon l'invention sont avantageusement les huiles siliconées telles que les polydiméthylsiloxanes (PDMS), liquides à température ambiante, volatils ou non, linéaires ou cycliques ; les polydiméthylsiloxanes comportant des groupements alkyle ou alcoxy, pendant ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, leurs mélanges ; les hydrocarbures ou fluorocarbures linéaires ou ramifiés d'origine synthétique ou minérale, volatils ou non comme les huiles de paraffine et leurs dérivés (telles que les isoparafines et l'isododécane), l'huile de vaseline, les polydécènes, le polyisobutène hydrogéné tel que le Parléam, le squalane et leurs mélanges. De préférence, les huiles structurées, et plus spécialement celles structurées par les polyamides et en particulier ceux de formules (I) ou les polyuréthanes, sont des huiles apolaires et plus spécialement une huile ou un mélange d'huiles, du type hydrocarbonées d'origine minérale ou synthétique, choisies en particulier parmi les hydrocarbures et notamment les alcanes comme le Parléam, le squalane, les isoparaffines et leurs mélanges. Avantageusement, ces huiles sont associées à une ou plusieurs huiles de silicones phénylées.

La phase grasse liquide représente, en pratique, de 5 à 99 % du poids total de la composition, de préférence de 10 à 80 % et mieux de 20 à 75 % .

La composition selon l'invention contient, en outre, un corps gras pâteux à savoir un produit visqueux contenant une fraction liquide et une fraction solide. Par « corps gras pâteux » au sens de l'invention, on entend des corps gras ayant un point de fusion allant de 20 à 55 °C, de préférence 25 à 45 °C, et/ou une viscosité à 40 °C allant de 0,1 à 40 Pa.s (1 à 400 poises), de préférence 0,5 à 25 Pa.s, mesurée au Contraves TV, équipé d'un mobile tournant à 240 min⁻¹. L'homme du métier peut choisir le mobile permettant de mesurer la viscosité, parmi les mobiles MS-r3 et MS-r4, sur la base de ses connaissances générales, de manière à pouvoir réaliser la mesure de la viscosité du composé pâteux testé.

Les valeurs de point de fusion correspondent, selon l'invention, au pic de fusion mesurée par la méthode "Differential Scanning Calorimetry" avec une montée en température de 5 ou 10 °C/min.

Selon l'invention, on utilise un ou plusieurs corps gras pâteux. De préférence, ces corps gras sont des composés hydrocarbonés (contenant principalement des atomes de carbone et d'hydrogène et éventuellement des groupements ester), éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le "THIXINR ®" de Rheox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le ou les corps gras pâteux peuvent être présents à raison de 0,5 à 60% en poids, par rapport au poids total de la composition, de préférence à raison de 2-45% en poids et encore plus préférentiellement à raison de 5-30% en poids, dans la composition.

On a de plus constaté que la composition selon l'invention permettait l'obtention d'un film de coloration homogène, en présence de matière colorante, dû à un bon mouillage des pigments par les corps gras pâteux. De plus, le film obtenu est facilement applicable et s'étale facilement sur le support. Il présente également une texture légère et est très confortable à porter tout au long de la journée.

La composition de l'invention peut comprendre, en outre, tout additif usuellement utilisé dans le domaine concerné, choisi notamment parmi l'eau éventuellement épaissie ou gélifiée par un épaississant ou un gélifiant de phase aqueuse, les antioxydants, les huiles essentielles, les conservateurs, les parfums, les charges, les cires, les neutralisants, les dispersants, les polymères liposolubles, les actifs cosmétiques ou dermatologiques comme par exemple des émollients, des hydratants, des vitamines, des acides gras essentiels, des filtres solaires, et leurs mélanges. Ces additifs peuvent être présents dans la composition à raison de 0 à 30% du poids total de la composition et mieux de 0,01 à 20% (si présents).

Avantageusement, la composition contient au moins un actif cosmétique ou dermatologique et/ou au moins une matière colorante.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut se présenter sous la forme d'une composition teintée dermatologique ou de soin des matières kératiniques comme la peau, les lèvres et/ou les phanères, sous forme d'une composition de protection solaire ou d'hygiène corporelle notamment sous forme de produit déodorant ou démaquillant en particulier sous forme de stick. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux). Elle se présente alors, généralement sous forme non colorée et renferme un ou plusieurs actifs. et/ou du vent, crème de soin pour la peau, les ongles ou les cheveux). Elle se présente alors, généralement sous forme non colorée et renferme un ou plusieurs actifs.

La composition de l'invention peut également se présenter sous la forme d'un produit coloré de maquillage de la peau, présentant éventuellement des propriétés de soin ou de traitement, et en particulier un fond de teint, un blush, un fard à joues ou à paupières, un produit anti-cerne, un eye-liner, un produit de maquillage du corps ; de maquillage des lèvres comme un rouge à lèvres, présentant éventuellement des propriétés de soin ou de traitement ; de maquillage des phanères comme les ongles, les cils en particulier sous forme d'un mascara, les sourcils et les cheveux notamment sous forme de crayon.

Bien entendu la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains. Par cosmétiquement acceptable, on entend au sens de l'invention une composition d'aspect, d'odeur et de toucher agréables.

Avantageusement, la composition contient une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 50 % du poids total de la composition, de préférence de 0,5 à 40 % et mieux de 5 à 25 %, si elle est présente.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine. Ils peuvent représenter de 0 à 20 % du poids de la composition et mieux de 0,1 à 6 % (si présents). Les colorants hydrosolubles sont par exemple le jus de betterave, le bleu de méthylène, et peuvent représenter jusqu'à 6 % du poids total de la composition.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0 à 50 %, de préférence de 0,5 à 40 % et mieux de 2 à 25 % du poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 %.

La composition peut éventuellement contenir au moins une cire ou un mélange de cires. Une cire, au sens de la présente invention, est un composé gras lipophile, solide à température ambiante (25°C) et pression atmosphérique (760mm de Hg), à changement d'état solide/liquide réversible, ayant une température de fusion supérieure à 40°C et mieux supérieure à 55 °C et pouvant aller jusqu'à 200° C, et présentant à l'état solide une moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires, au sens de la demande, sont celles généralement utilisées dans les domaines cosmétique et dermatologique; elles sont notamment d'origine naturelle comme la cire d'abeilles éventuellement modifiée, la cire de Carnauba, de Candelilla, d'Ouricoury, du Japon, de fibres de liège ou de canne à sucre, les cires de paraffine, de lignite, les cires microcristallines, la cire de Montan, les ozokérites, les huiles hydrogénées comme l'huile de jojoba hydrogénée, mais aussi d'origine synthétique comme les cires de polyéthylène issues de la polymérisation de l'éthylène, les cires obtenues par synthèse de Fischer-Tropsch, les esters d'acldes gras et les glycérides concrets à 40°C et mieux à plus de 55°C, les cires de silicone comme les alkyle, alcoxy et/ou esters de poly(di)méthylsiloxane solides à 40°C et mieux à plus de 55°C.

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le ou les composés amphiphiles, les matières colorantes et les additifs puis à mélanger le tout jusqu'à l'obtention d'une solution claire, transparente. Le mélange homogène obtenu peut alors être coulé dans un moule approprié comme un moule de rouge à lèvres ou directement dans les articles de conditionnement (boîtier ou coupelle notamment).

L'invention a encore pour objet un procédé cosmétique de soin, de maquillage ou de traitement des matières kératiniques des êtres humains et notamment de la peau, des lèvres et des phanères, comprenant l'application sur les matières kératiniques de la composition notamment cosmétique telle que définie ci-dessus.

L'invention a aussi pour objet l'utilisation d'une quantité suffisante d'au moins un corps gras pâteux ayant un point de fusion allant de 20 à 55 °C, et/ou une viscosité à 40 °C allant de 0,1 à 40 Pa.s mesurée au Contraves TV, équipé d'un mobile tournant à 240 min⁻¹, et d'au moins un polymère de masse moléculaire moyenne en poids inférieure à 100 000 et mieux inférieure à 50 000, comportant: a) un squelette polymérique, ayant des motifs de répétition hydrocarbonés pourvus d'au moins un hétéroatome, et b) éventuellement au moins une chaîne grasse pendante et/ou au moins une chaîne terminale, éventuellement fonctionnalisées ayant de 8 à 120 et notamment de 12 à 120 atomes de carbone, liées à ces motifs, ces chaînes grasses représentant de 40 à 98 % du nombre total des motifs à hétéroatome et des chaînes grasses, dans une composition cosmétique ou pour la fabrication d'une composition physiologiquement acceptable, contenant une phase grasse liquide, pour conférer à ladite composition des propriétés de longue tenue.

L'invention a encore pour objet l'utilisation d'une phase grasse liquide, structurée essentiellement par une quantité suffisante d'au moins un corps gras pâteux ayant un point de fusion allant de 20 à 55 °C, et/ou une viscosité à 40 °C allant de 0,1 à 40 Pa.s mesurée au Contraves TV, équipé d'un mobile tournant à 240 min⁻¹, et d'au moins un polymère de masse moléculaire moyenne en poids inférieure à 100 000 et mieux inférieure à 50 000, comportant a) un squelette polymérique, ayant des motifs de répétition hydrocarbonés pourvus d'au moins un hétéroatome, et b) éventuellement au moins une chaîne grasse pendante et/ou au moins une chaîne terminale, éventuellement fonctionnalisées ayant de 8 à 120 et en particulier de 12 à 120 atomes de carbone, liées à ces motifs, ces chaînes grasses représentant de 40 à 98 % du nombre total des motifs à hétéroatome et des chaînes grasses, dans une composition cosmétique ou pour la à hétéroatome et des chaînes grasses, dans une composition cosmétique ou pour la fabrication d'une composition physiologiquement acceptable brillante et/ou non migrante et/ou de longue tenue.

De préférence, le polymère a une masse moléculaire moyenne en poids allant de 1 000 à 30 000 et mieux de 1 000 à 10 000.

L'obtention d'une composition de bonne tenue, brillante et/ou non migrante grâce à l'association d'au moins un corps gras pâteux et d'au moins un polymère de masse moléculaire moyenne en poids inférieur à 100 000, concerne aussi des polymères ne comportant pas de chaîne pendante ou de chaîne terminale. Ces polymères sont en particulier des polyurées, des polyuréthanes et des polyamides structurant de phase grasse et plus spécialement des polyamides tels que ceux décrits dans le document US-A-3148125.

L'invention est illustrée plus en détail dans les exemples suivants. Les quantités sont données en pourcentage massique.

### Exemple 1 : Rouge à lèvres

- Uniclear 80 18,00 %
- Huile de parléam 26,07 %
- Octyldodécanol 9,00 %
- Acide poly(12-hydroxystéarique) 2,00 %
- Pigments 8.66 %
- Lanoline (pâteux) 5,00 %
- Cires 3,00 %
- Charges 3,00 %
- Silicone phénylée (huile) 5,00 %

*Préparation* : On solubilise (ou dissous) l'Uniclear 80 grâce à l'octyldodécanol dans l'huile de parléam, à 100 °C, puis on ajout les pigments et les charges. On ajoute les cires, et les huiles préalablement fondues à 90 °C. L'ensemble est mélangé à l'aide d'une turbine défloculeuse (Raynerie) puis coulé dans des moules de rouge à lèvres.

Le rouge à lèvres obtenu est brillant, non migrant et de bonne tenue. Ceci a été confirmé par un test sur un panel d'experts en comparaison avec un produit brillant de l'art antérieur Rouge Absolu de Lancôme. En effet, pour une brillance et un confort comparable, le rouge à lèvres de l'invention tient beaucoup mieux dans le temps et en particulier en ce qui concerne sa couleur. En outre, il a été jugé plus brillant qu'un produit longue tenue de l'art antérieur comme Rouge Magnetic et plus confortable.

## Revendications

1. Composition structurée contenant une phase grasse liquide, structurée par au moins un corps gras pâteux et au moins un polymère de masse moléculaire moyenne en poids inférieure à 100 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées ayant de 8 à 120 atomes de carbone, liées à ces motifs, les chaînes grasses représentant de 40 à 98 % du nombre total des motifs à hétéroatome et des chaînes grasses, la phase grasse liquide, le corps gras pâteux et le polymère formant un milieu physiologiquement acceptable, le corps gras pâteux ayant un point de fusion allant due 20 à 55 °C, et/ou une viscosité à 40 °C allant de 0,1 à 40 Pa.s mesurée au Contraves TV, équipé d'un mobile tournant à 240 min⁻¹.

2. Composition selon la revendication 1, **caractérisée en ce que** les chaînes grasses représentent de 50 à 95 % du nombre total des motifs à hétéroatome et des chaînes grasses.

3. Composition selon la revendication 1 ou 2, **caractérisée en ce que** les motifs à hétéroatome sont des amides.

4. Composition selon l'une des revendications précédentes, **caractérisée en ce que** les chaînes grasses pendantes sont liées directement à l'un au moins desdits hétéroatomes.

5. Composition selon l'une des revendications précédentes, **caractérisée en ce que** les chaînes grasses terminales sont liées au squelette par des groupes ester.

6. Composition selon l'une des revendications précédentes, **caractérisée en ce que** les chaînes grasses ont de 12 à 120 et mieux de 12 à 68 atomes de carbone.

7. Composition selon l'une des revendications précédentes, **caractérisée en ce que** le polymère est choisi parmi les polymères de formule (l) suivante et leurs mélanges : 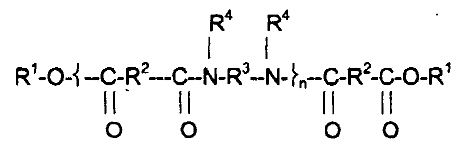 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

8. Composition selon la revendication précédente, **caractérisée en ce que** R¹ est un groupe alkyle en C₁₂ à C₂₂ et de préférence en C₁₆ à C₂₂.

9. Composition selon l'une des revendications 7 ou 8, **caractérisée en ce que** R² est un groupe ayant de 30 à 42 atomes de carbone.

10. Composition selon l'une des revendications 7 à 9, **caractérisée en ce que** R₃ est un groupe hydrocarboné en C₂ à C₃₆.

11. Composition selon l'une des revendications précédentes, **caractérisée en ce que** le polymère présente une masse moléculaire moyenne en poids allant de 1 000 à 30 000 et mieux de 1 000 à 10 000.

12. Composition selon l'une des revendications précédentes, **caractérisée en ce que** la composition comprend, en outre, au moins un composé amphiphile liquide à température ambiante, de valeur de HLB inférieure à 12 et mieux inférieure à 8 et notamment allant de 1 à 7 et de préférence de 1 à 5.

13. Composition selon la revendication précédente, **caractérisée en ce que** le composé amphiphile comprend une partie lipophile liée à une partie polaire, la partie lipophile comportant une chaîne carbonée ayant au moins 8 atomes de carbone, de préférence de 16 à 32 atomes de carbone et mieux de 18 à 28 atomes de carbone.

14. Composition selon la revendication précédente, **caractérisée en ce que** la partie polaire est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés.

15. Composition selon l'une des revendications 11 à 13, **caractérisée en ce que** le composé amphiphile est choisi parmi les hydroxystéarates, les oléates, les isostéarates du glycérol, du sorbitan ou du méthylglucose, l'octyldodécanol.

16. Composition selon l'une des revendications 11 à 14, **caractérisée en ce que** le composé amphiphile représente de 0,1 à 35 % du poids total de la composition.

17. Composition selon l'une des revendications précédentes, **caractérisée en ce que** le polymère représente de 0,5 à 80 % du poids total de la composition et mieux de 5 à 40 %.

18. Composition selon l'une des revendications précédentes, **caractérisée en ce que** la phase grasse contient une huile hydrocarbonée d'origine minérale ou synthétique.

19. Composition selon l'une des revendications précédentes, **caractérisée en ce que** la phase grasse contient une huile apolaire choisie parmi l'huile de parléam, le squalane et leurs mélanges.

20. Composition selon l'une des revendications précédentes, **caractérisée en ce que** la phase grasse contient une silicone phénylée.

21. Composition selon l'une des revendications précédentes, **caractérisée en ce que** la phase grasse liquide représente de 5 à 99 % du poids total de la composition et mieux de 20 à 75 %.

22. Composition selon l'une des revendications précédentes, **caractérisée en ce que** le corps gras pâteux présente une viscosité à 40°C de 0,5 à 25 Pa.s et/ou un point de fusion de 25 à 45°C.

23. Composition selon l'une des revendications précédentes, **caractérisée en ce que** le corps gras pâteux est choisi parmi les lanolines, les dérivés de lanoline ; les esters d'acides ou d'alcools gras, notamment ceux ayant 20 à 65 atomes de carbone ; le propionate d'arachidyle ; le polylaurate de vinyle ; les esters du cholestérol comme les triglycérides d'origine végétale ; les polyesters visqueux ; les corps gras pâteux siliconés comme les polydiméthylsiloxanes ayant des chaînes pendantes du type alkyle ou alcoxy ayant de 8 à 24 atomes de carbone et un point de fusion de 20 à 55°C, et leurs mélanges.

24. Composition selon l'une des revendications précédentes, **caractérisée en ce que** le corps gras pâteux est présent à raison de 0,5 à 60% en poids, par rapport au poids total de la composition, de préférence de 2-45% en poids et encore plus préférentiellement de 5-30% en poids.

25. Composition selon l'une des revendications précédentes, **caractérisée en ce qu'**elle constitue une composition de soin et/ou de traitement et/ou de maquillage des matières kératiniques.

26. Composition selon l'une des revendications précédentes, **caractérisée en ce qu'**elle contient, en outre, au moins une matière colorante.

27. Composition selon la revendication précédente, **caractérisée en ce que** la matière colorante est choisie parmi les colorants lipophiles, les colorants hydrophiles, les pigments, les nacres et leurs mélanges.

28. Composition selon la revendication 26 ou 27 précédente, **caractérisée en ce que** la matière colorante est présente à raison de 0,01 à 50 % du poids total de la composition, de préférence de 5 à 25 %.

29. Composition selon l'une des revendications précédentes, **caractérisée en ce qu'**elle contient au moins un additif choisi parmi l'eau, les antioxydants, les huiles essentielles, les conservateurs, les neutralisants, les dispersants, les polymères liposolubles, les charges, les parfums, les actifs cosmétiques ou dermatologiques, les cires et leurs mélanges.

30. Composition selon l'une des revendications précédentes, comprenant au moins une cire.

31. Composition selon l'une des revendications précédentes, **caractérisée en ce qu'**elle se présente sous forme d'un gel rigide, notamment de stick anhydre.

32. Composition selon l'une des revendications précédentes, **caractérisée en ce qu'**elle se présente sous forme de mascara, d'eye liner, de fond de teint, de rouge à lèvres, de blush, de produit déodorant ou démaquillant, de produit de maquillage du corps, de fard à paupières ou à joues, de produit anti-cerne, de shampooing, d'après-shampooing, de composition de protection solaire, de produit de soin du visage et du corps.

33. Procédé cosmétique de soin, de maquillage ou de traitement des matières kératiniques des êtres humains, comprenant l'application sur les matières kératiniques d'une composition cosmétique conforme à l'une des revendications précédentes.

34. Utilisation d'une quantité suffisante d'au moins un corps gras pâteux ayant un point de fusion allant de 20 à 55 °C, et/ou une viscosité à 40 °C allant de 0,1 à 40 Pa.s mesurée au Contraves TV, équipé d'un mobile tournant à 240 min⁻¹, et d'au moins un polymère de masse moléculaire moyenne en poids inférieure à 100 000, comportant a) un squelette polymérique ayant des motifs de répétition hydrocarbonés pourvus d'au moins un hétéroatome, et b) au moins une chaîne grasse pendante et/ou au moins une chaîne terminale, éventuellement fonctionnalisées ayant de 8 à 120 atomes de carbone, liées à ces motifs, ces chaînes grasses représentant de 40 à 98 % du nombre total des motifs à hétéroatome et des chaînes grasses, dans une composition cosmétique ou pour la fabrication d'une composition physiologiquement acceptable contenant une phase grasse, pour conférer à ladite composition des propriétés de longue tenue.

35. Utilisation d'une phase grasse liquide, structurée essentiellement par une quantité suffisante d'au moins un corps gras pâteux ayant un point de fusion allant de 20 à 55 °C, et/ou une viscosité à 40 °C allant de 0,1 à 40 Pa.s mesurée au Contraves TV, équipé d'un mobile tournant à 240 min⁻¹, et d'au moins un polymère de masse moléculaire moyenne en poids inférieure à 100 000 et mieux inférieure à 50 000, comportant a) un squelette polymérique, ayant des motifs de répétition hydrocarbonés pourvus d'au moins un hétéroatome, et b) éventuellement au moins une chaîne grasse pendante et/ou au moins une chaîne terminale, éventuellement fonctionnalisées ayant de 8 à 120 et en particulier de 12 à 120 atomes de carbone, liées à ces motifs, ces chaînes grasses représentant de 40 à 98 % du nombre total des motifs à hétéroatome et des chaînes grasses, dans une composition cosmétique ou pour la fabrication d'une composition physiologiquement acceptable brillante et/ou non migrante et/ou de longue tenue.

36. Utilisation selon la revendication 34 ou 35, **caractérisée en ce que** le polymère est un polyamide comportant des groupements terminaux à fonction ester comportant une chaîne hydrocarbonée ayant de 10 à 42 atomes de carbone.

37. Utilisation selon la revendication 34, 35 ou 36, **caractérisée en ce que** le polymère est associé à un composé amphiphile liquide à température ambiante, de valeur de HLB inférieure à 12 et mieux inférieure à 8 et mieux encore allant de 1 à 7 et de préférence de 1 à 5.

## Patentansprüche

1. Strukturierte Zusammensetzung, die eine flüssige Fettphase enthält, die mit mindestens einer pastösen Fettsubstanz und mindestens einem Polymer mit einem Gewichtsmittel der Molmasse unter 100 000 strukturiert ist, das a) ein Polymergerüst mit wiederkehrenden Kohlenwasserstoffeinheiten, die mindestens ein Heteroatom enthalten, und b) mindestens eine Fettkette als Seitenkette und/oder mindestens eine endständige Fettkette mit 8 bis 120 Kohlenstoffatomen aufweist, die gegebenenfalls funktionalisiert sind und die an diese Einheiten gebunden sind, wobei die Fettketten 40 bis 98 % der Gesamtzahl der Einheiten mit Heteroatom und der Fettketten ausmachen, wobei die flüssige Fettphase, die pastöse Fettsubstanz und das Polymer ein physiologisch akzeptables Medium bilden und die pastöse Fettsubstanz einen Schmelzpunkt von 20 bis 55 °C und/oder bei 40 °C eine Viskosität von 0,1 bis 40 Pa·s besitzt, die mit einem Viskosimeter Contraves TV gemessen wird, dessen bewegliches Teil bei 240 min⁻¹ arbeitet.

2. Zusammensetzung nach Anspruch 1, **dadurch gekennzeichnet, dass** die Fettketten 50 bis 95 % der Gesamtzahl der Einheiten mit Heteroatom und der Fettketten ausmachen.

3. Zusammensetzung nach Anspruch 1 oder 2, **dadurch gekennzeichnet, dass** die Einheiten mit Heteroatom Amide sind.

4. Zusammensetzung nach einem der vorhergehenden Ansprüche, **dadurch gekennzeichnet, dass** die als Seitenketten vorliegenden Fettketten direkt an mindestens eines der Heteroatome gebunden sind.

5. Zusammensetzung nach einem der vorhergehenden Ansprüche, **dadurch gekennzeichnet, dass** die endständigen Fettketten über Estergruppen an das Gerüst gebunden sind.

6. Zusammensetzung nach einem der vorhergehenden Ansprüche, **dadurch gekennzeichnet, dass** die Fettketten 12 bis 120 Kohlenstoffatome und besser 12 bis 68 Kohlenstoffatome aufweisen.

7. Zusammensetzung nach einem der vorhergehenden Ansprüche, **dadurch gekennzeichnet, dass** das Polymer unter den Polymeren der folgenden Formel (I) und deren Gemischen ausgewählt ist: 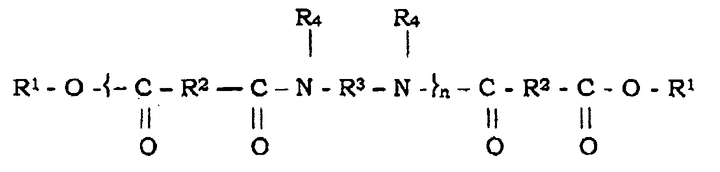 wobei n die Anzahl der Amideinheiten bezeichnet und so ausgewählt ist, dass die Anzahl der Estergruppen 10 bis 50 % der Gesamtzahl der Ester- und Amidgruppen ausmacht; die Gruppen R¹ jeweils unabhängig voneinander Alkyl- oder Alkenylgruppen mit mindestens 4 Kohlenstoffatomen bedeuten; die Gruppen R² jeweils unabhängig voneinander C₄₋₄₂-Kohlenwasserstoffgruppen bedeuten, mit der Maßgabe, dass mindestens 50 % der Gruppen R² C₃₀₋₄₂-Kohlenwasserstoffgruppen sind; die Gruppen R³ jeweils unabhängig voneinander organische Gruppen bedeuten, die mindestens zwei Kohlenstoffatome, Wasserstoffatome und Gewünschtenfalls ein oder mehrere Sauerstoffatome oder Stickstoffatome enthalten; und die Gruppen R⁴ jeweils unabhängig voneinander Wasserstoff, C₁₋₁₀-Alkyl oder eine direkte Bindung zu R³ oder einer weiteren Gruppe R⁴ bedeuten, die so gebildet wird, dass das Stickstoffatom, an das die Gruppen R³ und R⁴ gleichzeitig gebunden sind, Teil einer heterocyclischen Struktur ist, die durch R⁴-N-R³ definiert ist, wobei mindestens 50 % der Gruppen R⁴ Wasserstoff bedeuten.

8. Zusammensetzung nach dem vorhergehenden Anspruch, **dadurch gekennzeichnet, dass** R¹ eine C₁₂₋₂₂-Alkylgruppe und vorzugsweise eine C₁₆₋₂₂-Alkylgruppe ist.

9. Zusammensetzung nach einem der Ansprüche 7 oder 8, **dadurch gekennzeichnet, dass** R² eine Gruppe mit 30 bis 42 Kohlenstoffatomen ist.

10. Zusammensetzung nach einem der Ansprüche 7 bis 9, **dadurch gekennzeichnet, dass** R₃ eine Kohlenwasserstoffgruppe mit 2 bis 36 Kohlenstoffatomen ist.

11. Zusammensetzung nach einem der vorhergehenden Ansprüche, **dadurch gekennzeichnet, dass** das Polymer ein Gewichtsmittel der Molmasse von 1 000 bis 30 000 und besser 1 000 bis 10 000 aufweist.

12. Zusammensetzung nach einem der vorhergehenden Ansprüche, **dadurch gekennzeichnet, dass** die Zusammensetzung ferner mindestens eine bei Raumtemperatur flüssige, amphiphile Verbindung enthält, die einen HLB-Wert unter 12, besser unter 8 und insbesondere im Bereich von 1 bis 7 und vorzugsweise 1 bis 5 aufweist.

13. Zusammensetzung nach dem vorhergehenden Anspruch, **dadurch gekennzeichnet, dass** die amphiphile Verbindung einen lipophilen Bereich aufweist, der an einen polaren Bereich gebunden ist, wobei der lipophile Bereich eine kohlenstoffhaltige Kette mit mindestens 8 Kohlenstoffatomen und vorzugsweise 16 bis 32 Kohlenstoffatomen und besser 18 bis 28 Kohlenstoffatomen enthält.

14. Zusammensetzung nach dem vorhergehenden Anspruch, **dadurch gekennzeichnet, dass** der polare Bereich der Rest einer Verbindung ist, die unter den Alkoholen und Polyolen mit 1 bis 12 Hydroxygruppen und den Polyoxyalkylenen ausgewählt ist, die mindestens 2 Alkylenoxideinheiten aufweisen und 0 bis 20 Propylenoxideinheiten und/oder 0 bis 20 Ethylenoxideinheiten besitzen.

15. Zusammensetzung nach einem der Ansprüche 11 bis 13, **dadurch gekennzeichnet, dass** die amphiphile Verbindung unter den Hydroxystearaten, Oleaten, Isostearaten von Glycerin, Sorbitan oder Methylglucose und Octyldodecanol ausgewählt ist.

16. Zusammensetzung nach einem der Ansprüche 11 bis 14, **dadurch gekennzeichnet, dass** die amphiphile Verbindung 0,1 bis 35 % des Gesamtgewichts der Zusammensetzung ausmacht.

17. Zusammensetzung nach einem der vorhergehenden Ansprüche, **dadurch gekennzeichnet, dass** das Polymer 0,5 bis 80 % des Gesamtgewichts der Zusammensetzung und besser 5 bis 40 % ausmacht.

18. Zusammensetzung nach einem der vorhergehenden Ansprüche, **dadurch gekennzeichnet, dass** die Fettphase ein Kohlenwasserstofföl mineralischer oder synthetischer Herkunft enthält.

19. Zusammensetzung nach einem der vorhergehenden Ansprüche, **dadurch gekennzeichnet, dass** die Fettphase ein apolares Öl enthält, das unter Parleam, Squalan und deren Gemischen ausgewählt ist.

20. Zusammensetzung nach einem der vorhergehenden Ansprüche, **dadurch gekennzeichnet, dass** die Fettphase ein phenylgruppenhaltiges Silicon enthält.

21. Zusammensetzung nach einem der vorhergehenden Ansprüche, **dadurch gekennzeichnet, dass** die flüssige Fettphase 5 bis 99 % des Gesamtgewichts der Zusammensetzung und besser 20 bis 75 % ausmacht.

22. Zusammensetzung nach einem der vorhergehenden Ansprüche, **dadurch gekennzeichnet, dass** die pastöse Fettsubstanz bei 40 °C eine Viskosität von 0,5 bis 25 Pa·s und/oder einen Schmelzpunkt von 25 bis 45 °C ausweist.

23. Zusammensetzung nach einem der vorhergehenden Ansprüche, **dadurch gekennzeichnet, dass** die pastöse Fettsubstanz unter den Lanolinen und Lanolinderivaten; Estern von Fettsäuren oder Fettalkoholen, insbesondere Verbindungen mit 20 bis 65 Kohlenstoffatömen; Arachidylpropionat; Polyvinyllaurat; Cholesterinestern, wie Triglyceriden pflanzlicher Herkunft; viskosen Polyestern; siliconhaltigen pastösen Fettsubstanzen, wie Polydimethylsiloxanen, die Seitengruppen vom Alkyl- oder Alkoxytyp mit 8 bis 24 Kohlenstoffatomen enthalten und einen Schmelzpunkt von 20 bis 55 °C aufweisen, und deren Gemischen ausgewählt ist.

24. Zusammensetzung nach einem der vorhergehenden Ansprüche, **dadurch gekennzeichnet, dass** die pastöse Fettsubstanz in einer Menge von 0,5 bis 60 Gew.-%, bezogen auf das Gesamtgewicht der Zusammensetzung, vorzugsweise 2 bis 45 Gew.-% und noch bevorzugter 5 bis 30 Gew.-% enthalten ist.

25. Zusammensetzung nach einem der vorhergehenden Ansprüche, **dadurch gekennzeichnet, dass** sie eine Zusammensetzung zur Pflege und/oder zur Behandlung und/oder zum Schminken von Keratinsubstanzen ist.

26. Zusammensetzung nach einem der vorhergehenden Ansprüche, **dadurch gekennzeichnet, dass** sie ferner mindestens ein Farbmittel enthält.

27. Zusammensetzung nach dem vorhergehenden Anspruch, **dadurch gekennzeichnet, dass** das Farbmittel unter den lipophilen Farbstoffen, hydrophilen Farbstoffen, Pigmenten, Perlglanzpigmenten und deren Gemischen ausgewählt ist.

28. Zusammensetzung nach Anspruch 26 oder 27, **dadurch gekennzeichnet, dass** das Farbmittel in Mengenanteilen von 0,01 bis 40 % des Gesamtgewichts der Zusammensetzung und vorzugsweise 5 bis 25 % vorliegt.

29. Zusammensetzung nach einem der vorhergehenden Ansprüche, **dadurch gekennzeichnet, dass** sie mindestens einen Zusatzstoff enthält, der unter Wasser, Antioxidantien, etherischen Ölen, Konservierungsmitteln, Neutralisationsmitteln, Dispergiermitteln, fettlöslichen Polymeren, Füllstoffen, Parfums, kosmetischen oder dermatologischen Wirkstoffen, Wachsen und deren Gemischen ausgewählt ist.

30. Zusammensetzung nach einem der vorhergehenden Ansprüche, **dadurch gekennzeichnet, dass** sie mindestens ein Wachs enthält.

31. Zusammensetzung nach einem der vorhergehenden Ansprüche, **dadurch gekennzeichnet, dass** als starres Gel und insbesondere als wasserfreier Stift vorliegt.

32. Zusammensetzung nach einem der vorhergehenden Ansprüche, **dadurch gekennzeichnet, dass** sie als Mascara, Eyeliner, Makeup, Lippenstift, Rouge, Deodorant, Produkt zum Abschminken, Produkt zum Schminken des Körpers, Lidschatten, Wangenrouge, Produkt gegen Augenringe, Haarwaschmittel, Haarpflegemittel, das nach der Haarwäsche angewandt wird, Produkt zum Sonnenschutz und Produkt zur Pflege des Gesichts oder des Körpers vorliegt.

33. Kosmetisches Verfahren zur Pflege, zum Schminken oder zur Behandlung von menschlichen Keratinsubstanzen, das umfasst, auf die Keratinsubstanzen eine kosmetische Zusammensetzung nach einem der vorhergehenden Ansprüche aufzutragen.

34. Verwendung einer ausreichenden Menge mindestens einer pastösen Fettsubstanz, die einen Schmelzpunkt von 20 bis 55 °C und/oder bei 40 °C eine Viskosität von 0,1 bis 40 Pa·s besitzt, die mit einem Viskosimeter Contraves TV gemessen wird, dessen bewegliches Teil bei 240 min⁻¹ arbeitet, und mindestens eines Polymers mit einem Gewichtsmittel der Molmasse unter 100 000, das a) ein Polymergerüst mit wiederkehrenden Kohlenwasserstoffeinheiten, die mindestens ein Heteroatom enthalten, und b) mindestens eine Fettkette als Seitenkette und/ oder mindestens eine endständige Fettkette mit 8 bis 120 Kohlenstoffatomen aufweist, die gegebenenfalls funktionalisiert sind und die an diese Einheiten gebunden sind, wobei die Fettketten 40 bis 98 % der Gesamtzahl der Einheiten mit Heteroatom und der Fettketten ausmachen, in einer kosmetischen Zusammensetzung oder zur Herstellung einer physiologisch akzeptablen Zusammensetzung, die eine Fettphase enthält, um der Zusammensetzung Langzeitbeständigkeit zu geben.

35. Verwendung einer flüssigen Fettphase, die im Wesentlichen mit einer ausreichenden Menge mindestens einer pastösen Fettsubstanz, die einen Schmelzpunkt von 20 bis 55 °C und/ oder bei 40 °C eine Viskosität von 0,1 bis 40 Pa·s besitzt, die mit einem Viskosimeter Contraves TV gemessen wird, dessen bewegliches Teil bei 240 min⁻¹ arbeitet, und mindestens eines Polymers mit einem Gewichtsmittel der Molmasse unter 100 00 und besser unter 50 000 strukturiert wurde, das a) ein Polymergerüst mit wiederkehrenden Kohlenwasserstoffeinheiten, die mindestens ein Heteroatom enthalten, und b) mindestens eine Fettkette als Seitenkette und/oder mindestens eine endständige Fettkette mit 8 bis 120 Kohlenstoffatomen und insbesondere 12 bis 120 Kohlenstoffatomen aufweist, die gegebenenfalls funktionalisiert sind und die an diese Einheiten gebunden sind, wobei die. Fettketten 40 bis 98 % der Gesamtzahl der Einheiten mit Heteroatom und der Fettketten ausmachen, in einer kosmetischen Zusammensetzung oder zur Herstellung einer physiologisch akzeptablen Zusammensetzung, die glänzt und/oder keine Migration zeigt und/oder lange beständig ist.

36. Verwendung nach Anspruch 34 oder 35, **dadurch gekennzeichnet, dass** das Polymer ein Polyamid ist, das endständige Gruppen mit Esterfunktion aufweist, die eine Kohlenwasserstoffkette mit 10 bis 42 Kohlenstoffatomen umfassen.

37. Verwendung nach Anspruch 34, 35 oder 36, **dadurch gekennzeichnet, dass** das Polymer mit einer bei Raumtemperatur flüssigen, amphiphilen Verbindung kombiniert wird, die einen HLB-Wert unter 12, besser unter 8 und insbesondere im Bereich von 1 bis 7 und vorzugsweise 1 bis 5 aufweist.

## Claims

1. Structured composition comprising a liquid fatty phase, structured by at least one pasty fatty substance and at least one polymer with a weight-average molecular mass of less than 100 000, comprising a) a polymer backbone having hydrocarbonaceous repeat units provided with at least one heteroatom and b) at least one optionally functionalized pendent fatty chain and/or at least one optionally functionalized end fatty chain having from 8 to 120 carbon atoms bonded to these units, the fatty chains representing from 40 to 98% of the total number of the units with a heteroatom and of the fatty chains, the liquid fatty phase, the pasty fatty substance and the polymer forming a physiologically acceptable medium, the pasty fatty substance having a melting point ranging from 20 to 55°C and/or a viscosity at 40°C ranging from 0.1 to 40 Pa·s, measured on a Contraves TV equipped with a rotor rotating at 240 min⁻¹.

2. Composition according to Claim 1, **characterized in that** the fatty chains represent from 50 to 95% of the total number of the units with a heteroatom and of the fatty chains.

3. Composition according to Claim 1 or 2, **characterized in that** the units with a heteroatom are amides.

4. Composition according to one of the preceding claims, **characterized in that** the pendent fatty chains are bonded directly to at least one of the said heteroatoms.

5. Composition according to one of the preceding claims, **characterized in that** the end fatty chains are bonded to the backbone via ester groups.

6. Composition according to one of the preceding claims, **characterized in that** the fatty chains have from 12 to 120 and better still from 12 to 68 carbon atoms.

7. Composition according to one of the preceding claims, **characterized in that** the polymer is chosen from the polymers of following formula (I) and their mixtures: 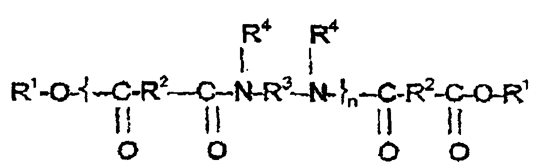 in which n denotes a number of amide units such that the number of ester groups represents from 10% to 50% of the total number of the ester and amide groups; R¹ is, in each instance, independently an alkyl or alkenyl group having at least 4 carbon atoms; R² independently represents, in each instance, a C₄ to C₄₂ hydrocarbonaceous group, provided that 50% of the R² groups represent a C₃₀ to C₄₂ hydrocarbonaceous group; R³ independently represents, in each instance, an organic group provided with at least 2 carbon atoms, with hydrogen atoms and optionally with one or more oxygen or nitrogen atoms; and R⁴ independently represents, in each instance, a hydrogen atom, a C₁ to C₁₀ alkyl group or a direct bond to R³ or to another R⁴, so that the nitrogen atom to which both R³ and R⁴ are bonded forms part of a heterocyclic structure defined by R⁴-N-R³, with at least 50% of the R⁴ groups representing a hydrogen atom.

8. Composition according to the preceding claim, **characterized in that** R¹ is a C₁₂ to C₂₂ and preferably C₁₆ to C₂₂ alkyl group.

9. Composition according to either of Claims 7 and 8, **characterized in that** R² is a group having from 30 to 42 carbon atoms.

10. Composition according to one of Claims 7 to 9, **characterized in that** R³ is a C₂ to C₃₆ hydrocarbonaceous group.

11. Composition according to one of the preceding claims, **characterized in that** the polymer exhibits a weight-average molecular mass ranging from 1 000 to 30 000 and better still from 1 000 to 10 000.

12. Composition according to one of the preceding claims, **characterized in that** the composition additionally comprises at least one amphiphilic compound which is liquid at ambient temperature and which has an HLB value of less than 12 and better still of less than 8 and in particular ranging from 1 to 7 and preferably from 1 to 5.

13. Composition according to the preceding claim, **characterized in that** the amphiphilic compound comprises a lipophilic part bonded to a polar part, the lipophilic part comprising a carbonaceous chain having at least 8 carbon atoms, preferably from 16 to 32 carbon atoms and better still from 18 to 28 carbon atoms.

14. Composition according to the preceding claim, **characterized in that** the polar part is the residue of a compound chosen from alcohols and polyols having from 1 to 12 hydroxyl groups or polyoxyalkylenes comprising at least two oxyalkylene units and having from 0 to 20 oxypropylene units and/or from 0 to 20 oxyethylene units.

15. Composition according to one of Claims 11 to 13, **characterized in that** the amphiphilic compound is chosen from hydroxystearates, oleates or isostearates of glycerol, of sorbitan or of methylglucose, or octyldodecanol.

16. Composition according to one of Claims 11 to 14, **characterized in that** the amphiphilic compound represents from 0.1 to 35% of the total weight of the composition.

17. Composition according to one of the preceding claims, **characterized in that** the polymer represents from 0.5 to 80% of the total weight of the composition and better still from 5 to 40%.

18. Composition according to one of the preceding claims, **characterized in that** the fatty phase comprises a hydrocarbonaceous oil of mineral or synthetic origin.

19. Composition according to one of the preceding claims, **characterized in that** the fatty phase comprises a nonpolar oil chosen from parleam oil, squalane and their mixtures.

20. Composition according to one of the preceding claims, **characterized in that** the fatty phase comprises a phenylated silicone.

21. Composition according to one of the preceding claims, **characterized in that** the liquid fatty phase represents from 5 to 99% of the total weight of the composition and better still from 20% to 75%.

22. Composition according to one of the preceding claims, **characterized in that** the pasty fatty substance exhibits a viscosity at 40°C of 0.5 to 25 Pa·s and/or a melting point of 25 to 45°C.

23. Composition according to one of the preceding claims, **characterized in that** the pasty fatty substance is chosen from lanolins or lanolin derivatives; esters of fatty acids or of fatty alcohols, in particular those having 20 to 65 carbon atoms; arachidyl propionate; poly(vinyl laurate); cholesterol esters, such as triglycerides of vegetable origin; viscous polyesters; silicone pasty fatty substances, such as polydimethylsiloxanes having pendent chains of the alkyl or alkoxy type having from 8 to 24 carbon atoms and a melting point of 20 to 55°C, and their mixtures.

24. Composition according to one of the preceding claims, **characterized in that** the pasty fatty substance is present in a proportion of 0.5 to 60% by weight with respect to the total weight of the composition, preferably of 2-45% by weight and more preferably still of 5-30% by weight.

25. Composition according to one of the preceding claims, **characterized in that** it constitutes a composition for caring for and/or for treating and/or for making up keratinous substances.

26. Composition according to one of the preceding claims, **characterized in that** it additionally comprises at least one colouring material.

27. Composition according to the preceding claim, **characterized in that** the colouring material is chosen from lipophilic dyes, hydrophilic dyes, pigments, pearlescent agents and their mixtures.

28. Composition according to the preceding Claim 26 or 27, **characterized in that** the colouring material is present in a proportion of 0.01 to 50% of the total weight of the composition, preferably of 5 to 25%.

29. Composition according to one of the preceding claims, **characterized in that** it comprises at least one additive chosen from water, antioxidants, essential oils, preservatives, neutralizing agents, dispersing agents, fat-soluble polymers, fillers, fragrances, cosmetic or dermatological active principles, waxes and their mixtures.

30. Composition according to one of the preceding claims, comprising at least one wax.

31. Composition according to one of the preceding claims, **characterized in that** it is provided in the form of a stiff gel, in particular of an anhydrous stick.

32. Composition according to one of the preceding claims, **characterized in that** it is provided in the form of a mascara, an eyeliner, a foundation, a lipstick, a blusher, a deodorant or make-up removing product, a product for making up the body, an eyeshadow, a face powder, a concealer, a shampoo, a conditioner, an antisun composition, or a product for caring for the face and the body.

33. Cosmetic process for caring for, making up or treating the keratinous substances of human beings, comprising the application, to the keratinous substances, of a cosmetic composition in accordance with one of the preceding claims.

34. Use of a sufficient amount of at least one pasty fatty substance having a melting point ranging from 20 to 55°C and/or a viscosity at 40°C ranging from 0.1 to 40 Pa·s, measured on a Contraves TV equipped with a rotor rotating at 240 min⁻¹, and of at least one polymer with a weight-average molecular mass of less than 100 000, comprising a) a polymer backbone having hydrocarbonaceous repeat units provided with at least one heteroatom and b) at least one optionally functionalized pendent fatty chain and/or at least one optionally functionalized end chain having from 8 to 120 carbon atoms which are bonded to these units, these fatty chains representing from 40 to 98% of the total number of the units with a heteroatom and of the fatty chains, in a cosmetic composition or for the manufacture of a physiologically acceptable composition comprising a fatty phase, for conferring long-lasting properties on the said composition.

35. Use of a liquid fatty phase essentially structured by a sufficient amount of at least one pasty fatty substance having a melting point ranging from 20 to 55°C and/or a viscosity at 40°C ranging from 0.1 to 40 Pa·s, measured on a Contraves TV equipped with a rotor rotating at 240 min⁻¹, and of at least one polymer with a weight-average molecular mass of less than 100 000 and better still of less than 50 000, comprising a) a polymer backbone having hydrocarbonaceous repeat units provided with at least one heteroatom and b) optionally at least one optionally functionalized pendent fatty chain and/or at least one optionally functionalized end chain having from 8 to 120 and in particular from 12 to 120 carbon atoms which are bonded to these units, these fatty chains representing from 40 to 98% of the total number of the units with a heteroatom and of the fatty chains, in a cosmetic composition or for the manufacture of a physiologically acceptable composition which is glossy and/or which does not migrate and/or which has lengthy hold.

36. Use according to Claim 34 or 35, **characterized in that** the polymer is a polyamide comprising end groups with an ester functional group comprising a hydrocarbonaceous chain having from 10 to 42 carbon atoms.

37. Use according to Claim 34, 35 or 36, **characterized in that** the polymer is used in combination with an amphiphilic compound which is liquid at room temperature and which has an HLB value of less than 12 and better still of less than 8 and even better still ranging from 1 to 7 and preferably from 1 to 5.
